# EUROPEAN PATENT APPLICATION

(11) **EP 3 838 282 A1**
(43) Date of publication of application: **23.06.2021**
(21) Application number: 19807088.0
(22) Date of filing: 23.05.2019
(51) Int. Cl.: A61K 36/185, A61K 36/282, A61K 36/53, A61K 36/38, A61K 36/704, A23L 33/105, A61P 11/00, A61K 36/13

(54) **COMPOSITION FOR ALLEVIATING ASTHMA OR CHRONIC OBSTRUCTIVE PULMONARY DISEASE BY USING EPILOBIUM PYRRICHOLOPHUM EXTRACT AND LIKE**

(30) Priority: 23.05.2018 KR 20180058465; 23.05.2018 KR 20180058466; 23.05.2018 KR 20180058467; 23.05.2018 KR 20180058468
(71) Applicant: Korea Food Research Institute, Wanju-gun, Jeollabuk-do 55365 (KR)
(72) Inventor: SHIN, Hee Soon, Jeonju-si Jeollabuk-do 54871 (KR); NAM, Young Do, Jeonju-si Jeollabuk-do 55063 (KR); PARK, So Lim, Jeonju-si Jeollabuk-do 54867 (KR); SEO, Dong Ho, Jeonju-si Jeollabuk-do 54864 (KR); SHIN, Dong Uk, Cheongju-si Chungcheongbuk-do 28380 (KR); EOM, Ji Eun, Jeonju-si Jeollabuk-do 54964 (KR); LEE, So Young, Jeonju-si Jeollabuk-do 54865 (KR); JUNG, Sun Young, Seoul 05750 (KR); CHOI, Dae Woon, Taebaek-si Gangwon-do 26043 (KR)
(74) Representative: Algemeen Octrooi- en Merkenbureau B.V.
(86) International application number: PCT/KR2019/006222
(87) International publication number: WO 2019/225995

(57) **Abstract**

Disclosed in the present invention is a composition for ameliorating asthma or chronic obstructive pulmonary disease by using *Epilobium pyrricholophum* extract and the like that: inhibit NETosis induced by phorbol myristate acetate (PMA); inhibit the expression of inflammatory cytokines IL-6 and IL-8 in H292 cells, which are the bronchial epithelial cell line and are stimulated by cigarette smoke extract (CSE), porcine pancreas elastase (PPE) and ultrafine dust that is 2.5 micrometers or less in diameter (PM2.5); inhibit the expression of inflammatory cytokine MIP2 (IL-8) that are also in MH-S cells, which are mouse alveolar macrophages and are activated by CES; reduce the number of neutrophils and the like in BALF during animal model experiment in which COPD is induced by using CES and PPE; and reduce, in BALF or lung tissues, the concentration of inflammatory cytokines such as MIP-2, TNF-α, IL-1β, IL-6, KC, MCP-1 and IFN-γ and the concentration of MDC, GM-CSF, IL-17 and the like.

## Description

### [TECHNICAL FIELD]

The present invention relates to a composition for ameliorating asthma or chronic obstructive pulmonary diseases using *Epilobium pyrricholophum* extract and the like. More particularly, the present invention relates to a composition for ameliorating asthma or chronic obstructive pulmonary diseases using *Epilobium pyrricholophum* extract, *Hypericum erectum* extract, *Podocarpus macrophyllus* extract, *Artemisia gmelinii* extract, *Poria cocos* extract or *Elsholtzia ciliate* extract.

### [BACKGROUND ART]

Chronic obstructive pulmonary disease (COPD) is a chronic airway disease that shows irreversible airway obstruction, such as cough, sputum, dyspnea, decreased expiratory flow, and impaired gas exchange, and the number of affected people is increasing worldwide every year. This disease has been predicted to be the third cause of human death in 2020 (Am J Respir Crit Care Med, 2013, 187:347-365; Am J Respir Crit Care Med, 2009, 180:396-406). In the past, COPD was classified into chronic bronchitis and emphysema, but chronic bronchitis is defined on the basis of clinical symptoms, and emphysema is classified by anatomical criteria. Thus, in many cases, the same patient has both at the same time, and it is clinically difficult to distinguish them, so the diagnosis is made collectively as COPD (Eur Respir J, 2007, 30:993-1013; Respirology 1997, 2 Suppl 1:S1-4). In the case of bronchial asthma, when the duration of asthma becomes long and the airway obstruction shows irreversible changes, it should be included in COPD and treated in a similar manner (Eur Respir J, 2007, 30:993-1013; Respirology 1997, 2 Suppl 1:S1-4).

COPD is caused by a variety of causes, such as smoking, air pollution, chemical substances, occupational factors, and genetic predisposition, among which smoking is pointed out as the main cause. In fact, 80% or more of COPD patients have been found to be smokers (Biol Pharm Bull, 2012, 35:1752-1760). The pathogenic mechanism of COPD involves chronic inflammation in the bronchi and lung tissue, activation of proteolytic enzymes in the lung, oxidative stress and the like, and cells involved in inflammation are mainly neutrophils, macrophages, and T lymphocytes. These inflammatory cells produce various inflammatory cytokines such as TNF-α, IFN-γ, IL-1β, IL-6, IL-8, and IL-18, and various proteolytic enzymes that cause tissue damage (Korean Academy of Tuberculosis and Respiratory Disease, Respiratory Science, Seoul: Gunja publishing co.; 2007, p 301-5; Am J Respir Crit Care Med, 1997 155, 1441-1447; Am J Physiol Lung Cell Mol Physiol, 2010, 298:L262-L269). In the animal model of COPD induced by tobacco smoke, it was observed that the influx of neutrophils, KC(keratinocyte chemoattractant), TNF-α(tumor necrosis factorα), MIP-2(macrophage inflammatory protein 2), MIP-1α and MCP-1(monocyte chemoattractant protein), MMP12(matrix metalloproteinase 12) and GM-CSF (granulocyte macrophage colony-stimulating factor) were increased (Clin. Sci. 2014, 126(3):207).

COPD is not considered as a TH2-type disease such as atopy or asthma, but it has been reported that TH1 cells and TH2 cells are activated in COPD patients. Chemokine TARC (CCL17) and MDC (CCL22) are ligands of the chemokine receptor CCR4 expressed in TH2 cells (Curr. Opin. Immunol. 2002. 14: 129-135), and it has been reported that in asthma and COPD, TARC (CCL17) and MDC (CCL22) are increased together with TSLP (thymic stromal lymphopoietin), and in clinical trials, TARC (CCL17) and MDC (CCL22) are significantly increased in the smoking group compared to the non-smoking group (J Immunol 2008, 181(4):2790-8; Clin Exp Allergy 2012, 42:994-1005). Accordingly, reduction of TARC (CCL17) and MDC (CCL22) may be an important evaluation index associated with the amelioration of COPD.

Further, it was observed that the concentration of IL-17 in sputum and serum increased in COPD patients as in asthma patients, which has suggested that IL-17 plays a certain role in COPD. Even in the COPD animal model, IL-17 plays a decisive role in airway fibrosis, which has thereby proposed as an important target for the inhibition of airway fibrosis of COPD (Int J Chron Obstruct Pulmon Dis. 2017; 12:1247-1254; Chest. 2010 Nov; 138(5): 1140-7).

Meanwhile, it is known that neutrophils secrete substances such as extracellula elastase, collagenase, proteases such as MPO (myeloperoxidase), arachidonate metabolites, and reactive oxygen free radicals to cause lung damage, and cause chronic airway inflammation through airway infiltration, thereby playing a very important role in the onset of COPD (Am J Physiol Lung Cell Mol Physiol. 2017, 312(1):L122-L130; Am J Respir Cell Mol Biol, 2013, 48:531-539; Eur Respir J, 1998, 12:1200-1208).

Recently, it has been reported that excessive formation of neutrophil extracellular traps (NET) is associated with pulmonary diseases including COPD (PLoS One. 2014 May 15;9(5):e97784.; Respir Res. 2015 May 22;16:59.; J Immunol Res. 2017;2017:6710278; Respirology. 2016 Apr;21(3):467- 75). NET is a structure in the form of a net in which cytoplasmic proteins, granular proteins and the like are bound to chromatin intertwined with each other. NET captures and neutralizes bacteria, fungi, viruses and the like, and plays a role in preventing their spread and infection (Nat Immunol 15, 1017-1025, 2014), but excessive NET secretion has the association with various infectious and non-infectious diseases. In particular, since NET easily expands in an alveoli and induces lung damage, its association with lung disease has attracted attention (Nat Rev Microbiol. 2007, 5:577-82; Front Immunol. 2013, 4:1). Excessive NET formation (NETosis) has been reported in lung diseases such as, not only COPD but also lung diseases such as asthma, cystic fibrosis, respiratory syncytial virus (RSV) bronchiolitis, influenza virus infection, bacterial pneumonia, tuberculosis, transfusion-related acute lung injury. Therefore, the inhibition of NETosis is recognized as an important target for the development of treatments for these lung diseases (Front Immunol. 2016, 7:311).

Currently, bronchoalveolar lavage (BAL) is used as a means for observing the diagnosis and progress of diseases such as COPD and asthma, but in the bronchoalveolar lavage fluid (BALF) of these patients, inflammatory mediators such as inflammatory cytokines, reactive oxygen species, leukotriene, and activated complement are increased, and neutrophils, which account for less than 5% of normal lungs, increase to an extent that accounts for 80% of all cells (Am J Respir Crit Care Med 154(1):76-81, 1996).

So far, no drugs that directly ameliorate COPD or asthma have been reported. Current therapeutic drug of COPD or asthma are for reducing symptoms and complications, and bronchodilators (β2-agonists, anticholinergics, methylxanthines) and steroids (inhalation, oral) are mainly used.

The present invention discloses that *Epilobium pyrricholophum* extract and the like have the effect of ameliorating asthma or chronic obstructive pulmonary diseases.

### [DETAILED DESCRIPTION OF THE INVENTION]

### [Technical Problem]

An object of the present invention is to provide a composition for ameliorating asthma or chronic obstructive pulmonary diseases using *Epilobium pyrricholophum* extract and the like.

Other objects or specific objects of the present invention will be presented below.

### [Technical Solution]

As confirmed in the Examples and Experimental Examples described below, the present inventors have found that *Hypericum erectum* extract, *Podocarpus macrophyllus* extract, *Artemisia gmelinii* extract, *Poria cocos* extract or *Elsholtzia ciliate* extract and the like, including *Epilobium pyrricholophum* extract, inhibit NETosis induced by phorbol myristate acetate (PMA); or inhibit the expression of inflammatory cytokines IL-6 and IL-8 in H292 cells, which are the bronchial epithelial cell line and are stimulated by cigarette smoke extract (CSE), porcine pancreas elastase (PPE) and/or ultrafine dust that is 2.5 micrometers or less in diameter (PM2.5); or inhibit the expression of inflammatory cytokine MIP2 (IL-8) that are also in MH-S cells, which are mouse alveolar macrophages and are activated by CES; and reduce the number of neutrophils and the like in BALF during animal model experiment in which COPD is induced by using CES or, CES and PPE; and reduce, in BALF or lung tissues, the concentration of inflammatory cytokines such as MIP-2, TNF-α, IL-1β, IL-6, KC, MCP-1 and IFN-γ and the concentration of TARC(CCL17), MDC(CCL22), GM-CSF, IL-17 and the like.

The present invention is provided based on the experimental results described above. In one aspect of the present invention, there can be provided a composition for ameliorating asthma, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient. In other aspect, there can be provided a composition for ameliorating COPD, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient.
In another aspect, there can be provided a composition for ameliorating inflammatory lung diseases, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient. In another aspect of the present invention, there can be provided a composition for improving lung function and respiratory function, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient. In yet another aspect, there can be provided a composition for ameliorating respiratory diseases caused by smoking or fine dust, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient. In yet another aspect, there can be provided a composition for improving lung damage, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient. In a further aspect of the present invention, there can be provided a composition for ameliorating excressive NET formation, that is, lung diseases associated with NETosis, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient.

As used herein, the term "extract" refers to an extract obtained by leaching stems, leaves, fruits, flowers, roots, outposts of the plants to be extracted, and mixtures thereof using water, lower alcohols having 1 to 4 carbon atoms (methanol, ethanol, butanol, etc.), methylene chloride, ethylene, acetone, hexane, ether, chloroform, ethyl acetate, butyl acetate, N,N-dimethylformamide (DMF), dimethyl sulfoxide (DMSO), 1,3-butylene glycol, propylene glycol or a mixed solvent thereof, an extract obtained using supercritical extraction solvents such as carbon dioxide and pentane, or a fraction obtained by fractionating the extract. As the extraction method, any method such as cold extraction, reflux, heating, ultrasonic radiation, and supercritical extraction can be applied in consideration of the polarity of the active substance, extraction accuracy, and degree of preservation. The fractionated extract is meant to include a fraction obtained by suspending the extract in a specific solvent and then mixing and fixing it with a solvent of different polarity, a fraction obtained by adsorbing the crude extract on a column packed with silica gel or the like and then using a hydrophobic solvent, a hydrophilic solvent, or a mixed solvent thereof as a mobile phase. Further, the meaning of the extract includes a concentrated liquid extract or a solid extract from which the extraction solvent has been removed by a method such as freeze-drying, vacuum drying, hot air drying, spray drying, or the like. Preferably, the extract refers to an extract obtained by using water, ethanol or a mixed solvent thereof as an extraction solvent, more preferably an extract obtained by using a mixed solvent of water and ethanol as an extraction solvent.

Further, as used herein, the term "active ingredient" refers to an ingredient that exhibits a desired activity alone or can exhibit the activity together with a carrier that does not itself have an activity.

Further, as used herein, the term "inflammatory lung disease" is a lung disease associated with an inflammatory reaction and is meant to include asthma, chronic obstructive pulmonary disease (COPD), tracheitis, and bronchitis.

Further, as used herein, the term "improving lung function or respiratory function" refers to improving the breathing function of normal people which are non-patients, recovering the respiratory insufficiency in patients with chronic obstructive pulmonary disease, asthma, bronchitis, tracheitis, etc., or improving the respiratory function of patients with diseases.

Further, as used herein, the term "respiratory disease caused by smoking or fine dust" refers to including not only asthma and COPD, but also diffuse interstitial lung disease, acute respiratory distress syndrome (ARDS), and acute lung injury. The fine dust includes various ingredients, for example, carbon components such as soot and organic carbon of living organisms, ionic components such as chlorine, nitric acid, ammonium, sodium, and calcium, metal components such as lead, arsenic, and mercury, polycyclic aromatic hydrocarbons such as benzopyrene, and it is known that the fine dust deposits in the upper respiratory tract, bronchi, small airways, and alveoli to cause various lung diseases such as asthma and COPD (J Korean Med Assoc, 2014;57:763-768).

Further, as used herein, the term "improving lung damage" refers to recovery of lung cell or lung tissue damage caused by fine dust, etc. or recovery of deterioration in lung functions due to damage to lung cells or lung tissues.

As used herein, the "lung diseases associated with NETosis" is meant to include not only COPD but also asthma, cystic fibrosis, respiratory syncytial virus (RSV) bronchiolitis, influenza virus infection, bacterial pneumonia, tuberculosis, or transfusion-related acute lung injury.

Further, as used herein, the term "improvement" or "amelioration" is meant to include alleviation, treatment, or prevention of a disease or symptom.

In the composition of the present invention, the active ingredient may be included in an arbitrary amount (effective amount) according to the use, formulation, etc., as long as it can exhibit an effect of improving or ameliorating asthma, COPD, lung function or respiratory function, and the like. A typical effective amount will be determined within the range of 0.001% to 15% by weight based on the total weight of the composition. Here, the "effective amount" refers to the amount of the active ingredient contained in the composition of the present invention, that can produce the intended medical and pharmacological effects, such as ameliorating asthma, ameliorating COPD, and improving lung function or respiratory function, when the composition of the present invention is administered to a target mammal, preferably a human subject, during the administration period according to the advice of a medical professional or the like. Such effective amount can be determined empirically within the ordinary skill of a person skilled in the art.

In order to improve the effect of ameliorating asthma, ameliorating COPD, improving lung function or respiratory function, the composition of the present invention can, in addition to *Epilobium pyrricholophum* extract and the like as an active ingredient, further include *Curcuma longa* extract, *Scutellaria baicalensis* extract, *Trapa japonica* extract, *Perilla frutescens* extract, *Polygonum avivulare* extract, *Rosae multiflorea* extract, *Trigonella foenum* extract, *Piper nigrum* extract or a mixture of two or more thereof as an active ingredient, which are already known to have the effect of ameliorating asthma, ameliorating COPD, and improving lung function or respiratory function.

The composition of the present invention can be provided as a food composition in a specific embodiment.

The food composition of the present invention can be prepared in any form, and for example, can be prepared as beverages such as tea, juice, carbonated drinks, and ionized drinks, processed milk such as milk and yogurt, foods such as gum, rice cake, Korean confectionery, bread, sweets, and noodles, health functional food preparations such as tablets, capsules, pills, granules, liquids, powders, flakes, pastes, syrups, gels, jelly, bars, and the like. Further, the food composition of the present invention can take any product category as long as it conforms to the enforcement regulations at the time of manufacture and distribution in legal and functional category. For example, this composition may be a health functional food according to the Korean "Health Functional Food Act", or may be confectionery, beans, teas, beverages, special purpose foods, and the like based on the type of each food according to the Food Code of the Korean "Food Sanitation Act" ("Food Additive Standards and Specifications" notified by the Ministry of Food and Drug Safety).

The food composition of the present invention may contain food additives in addition to the active ingredients. The food additives can be generally understood as substances that are added to, mixed with, or infiltrated into foods in the manufacture, processing, or preservation of foods, but they are taken daily and for long period of time together with foods, so its safety must be guaranteed. In the Food Additive Code according to the laws of each country (in Korea, "Food Sanitation Act") governing the manufacture and distribution of foods, food additives whose safety is guaranteed are specified in terms of ingredients and functions in a limited manner. In the Korean Food Additives Code ("Food Additive Standards and Specifications" notified by the Ministry of Food and Drug Safety), food additives are classified into chemical synthetic products, natural additives, and mixed preparations in terms of ingredients, and these food additives are classified into sweeteners, flavoring agents, preservatives, emulsifiers, acidifiers, and thickening agents in terms of function.

The sweetener is used to impart a suitable sweetness to foods, and either natural sweetener or synthetic sweetener can be used in the food composition of the present invention, and the natural sweetener can be preferably used. Examples of the natural sweetener include sugar sweeteners such as corn syrup solid, honey, sucrose, fructose, lactose, and maltose.

The flavoring agent is used to improve taste or aroma, and both natural flavoring agent and synthetic flavoring agent can be used, and the natural flavoring agent can be preferably used. In the case of using natural ones, the purpose of nutrient enhancement can be combined in addition to flavor. The natural flavoring agent is obtained from apples, lemons, tangerines, grapes, strawberries, peaches, and the like, but it may be obtained from green tea leaves, solomon's seal, bamboo leaves, cinnamon, chrysanthemum leaves, jasmine, and the like. Further, those obtained from ginseng (red ginseng), bamboo shoots, aloe vera, ginkgo, and the like can be used. The natural flavoring agent may be a liquid concentrate or a solid extract. If necessary, the synthetic flavoring agent may be used. As the synthetic flavoring agent, esters, alcohols, aldehydes, terpenes, and the like may be used.

The preservatives that can be used include calcium sorbate, sodium sorbate, potassium sorbate, calcium benzoate, sodium benzoate, potassium benzoate, EDTA (ethylenediaminetetraacetic acid), and the like, the emulsifier may include acacia gum, carboxymethylcellulose, xanthan gum, pectin, and the like, and the acidifier may include citric acid, malic acid, fumaric acid, adipic acid, phosphoric acid, gluconic acid, tartaric acid, ascorbic acid, acetic acid, phosphoric acid, and the like. The acidifier can be added so that the food composition has an appropriate acidity for the purpose of suppressing the growth of microorganisms in addition to the purpose of enhancing taste. As the thickening agent, a suspending agent, a settling agent, a gel-forming agent, a swelling agent, and the like may be used.

The food composition of the present invention is a food additive known in the art for the purpose of supplementing and reinforcing functionality and nutrition, and in addition to the above-mentioned food additives, it may contain physiologically active substances or minerals whose stability is guaranteed.

Such bioactive substances may include catechins contained in green tea and the like, vitamins such as vitamin B1, vitamin C, vitamin E, and vitamin B12, tocopherol, dibenzoylthiamine, and the like, and the minerals may include calcium preparations such as calcium citrate, magnesium preparations such as magnesium stearate, iron preparations such as iron citrate, chromium chloride, potassium iodide, selenium, germanium, vanadium, zinc, and the like.

In the food composition of the present invention, the above-mentioned food additives may be included in an appropriate amount that can achieve the purpose of addition according to the type of product.

In connection with other food additives that can be included in the food composition of the present invention, the food code or food additive code according to the laws of each country may be referred to.

The composition of the present invention can be provided as a pharmaceutical composition in other specific embodiments.

The pharmaceutical composition of the present invention may include, in addition to the active ingredient, a pharmaceutically acceptable carrier, and may be prepared in an oral dosage form or parenteral dosage form depending on the route of administration by a conventional method known in the art. Here, the "pharmaceutically acceptable" means that it does not inhibit the activity of the active ingredient and a subject to be applied (prescribed) does not have toxicity above an applicable level.

When the pharmaceutical composition of the present invention is prepared in an oral dosage form, it can be prepared in a dosage form such as powders, granules, tablets, pills, dragee tablets, capsules, liquids, gels, syrups, suspensions, and wafers together with a suitable carrier in accordance with methods known in the art. At this time, examples of a suitable pharmaceutically acceptable carrier include sugars such as lactose, glucose, sucrose, dextrose, sorbitol, mannitol, and xylitol, starches such as corn starch, potato starch, wheat starch, celluloses such as cellulose, methyl cellulose, ethyl cellulose, sodium carboxymethyl cellulose, and hydroxypropyl methyl cellulose, polyvinyl pyrrolidone, water, methylhydroxybenzoate, propylhydroxybenzoate, magnesium stearate, mineral oil, malt, gelatin, talc, polyol, vegetable oil, and the like. In the case of formulation, it can be formulated including diluents and/or excipients such as fillers, extenders, binders, wetting agents, disintegrants, and surfactants, if necessary.

When the pharmaceutical composition of the present invention is prepared in a parenteral dosage form, it may be formulated in the form of eye drops, injections, transdermal administrations, nasal inhalations, or suppositories, together with a suitable carrier in accordance with methods known in the art. When formulated as an eye drop, suitable carriers may be sterile water, saline, or isotonic solutions such as 5% dextrose. If necessary, benzalkonium chloride, mefilparaben, ethylparaben, etc. may be added for preservative purposes. When formulated as an injection, a suitable carrier may include sterile water, ethanol, polyols such as glycerol or propylene glycol, or mixtures thereof. Preferably, Ringer's solution, phosphate buffered saline (PBS) containing triethanol amine, sterile water for injection, an isotonic solution such as 5% dextrose may be used. When formulated as a transdermal administration drug, it can be formulated in the form of an ointment, cream, lotion, gel, external solution, pasta, liniment, aerosol, and the like. For nasal inhalants, it can be formulated in the form of an aerosol spray using a suitable propellant such as dichlorofluoromethane, trichlorofluoromethane, dichlorotetrafluoroethane, carbon dioxide, and the like. When formulated as a suppository, the base may include witepsol, tween 61, polyethylene glycol, cacao butter, laurin paper, polyoxyethylene sorbitan fatty acid ester, polyoxyethylene stearate, sorbitan fatty acid ester, and the like.

The specific formulation of pharmaceutical compositions is known in the art, and for example, Remington's Pharmaceutical Sciences (19th ed., 1995) may be referred to, which is incorporated herein by reference.

The preferred dosage of the pharmaceutical composition of the present invention is in the range of 0.001 mg/kg to 10 g/kg per day, preferably 0.001 mg/kg to 1 g/kg per day, depending on the patient's condition, weight, sex, age, patient severity, and route of administration. Administration can be made once or divided into several times daily. Such dosage should not be construed as limiting the scope of the invention in any way.

### [ADVANTAGEOUS EFFECTS]

As described above, according to the present invention, a composition for ameliorating asthma or COPD using *Epilobium pyrricholophum* extract and the like can be provided. The composition of the present invention is commercialized as a food, especially a health functional food or a drug, and can be usefully used for asthma ameliorating effect or COPD ameliorating effect, further, for lung function or respiratory function improving effect.

### [BRIEF DESCRIPTION OF THE DRAWINGS]

FIG. 1 is the results showing the NETosis inhibitory activity of *Epilobium pyrricholophum* extract and the like.
FIGS. 2 to 4 are the results showing that *Epilobium pyrricholophum* extract has the inhibitory activity of inflammatory cytokines (IL-6 & IL-8) in H292 cells stimulated by CSE and the like.
FIG. 5 is the results showing that *Hypericum erectum* extract and the like has the inhibitory activity of inflammatory cytokines (IL-8) in H292 cells stimulated by CSE.
FIG. 6 is the result showing that *Epilobium pyrricholophum* extract and the like have the MIP-2 inhibitory activity using MH-S cells stimulated by CSE.
FIG. 7 is the result showing the Elastase inhibitory activity of *Hypericum erectum* extract and the like.
FIG. 8 is the result showing that *Epilobium pyrricholophum* extract and the like reduce the total number of infiltrating cells in BALF in an animal model experiment in which COPD is induced by CSE and PPE.
FIG. 9 is a result showing that *Epilobium pyrricholophum* extract and the like reduce the number of infiltrating immune cells in BALF in an animal model experiment in which COPD is induced by CSE and PPE.
FIGS. 10 to 12 are the results showing that *Epilobium pyrricholophum* extract and the like reduce the concentration of inflammatory cytokines or the like in BALF in an animal model experiment in which COPD is induced by CSE and PPE.
FIG. 13 is the result showing that *Podocarpus macrophyllus* extract reduces the concentration of inflammatory cytokines or the like in BALF in an animal model experiment in which COPD is induced by CSE and PPE.
FIGS. 14 to 16 are the results showing that *Artemisia gmelinii* extract lowers the concentration of BALF inflammatory cytokines or the like in an animal model experiment in which COPD is induced by CSE and PPE.
FIGS. 17 to 19 are the results showing that *Epilobium pyrricholophum* extract lowers the concentration of inflammatory cytokines or the like in lung tissue in an animal model experiment in which COPD is induced by CSE and PPE.
FIGS. 20 and 21 are the results showing that *Artemisia gmelinii* extract lowers the concentration of inflammatory cytokines in lung tissue in an animal model experiment in which COPD is induced by CSE and PPE.
FIG. 22 is the result showing that *Elsholtzia ciliate* extract reduces neutrophil infiltration in BALF and inhibits airway obstruction and alveolar destruction in an animal model experiment in which COPD is induced by CSE and PPE.
FIG. 23 is the result showing that *Poria cocos* extract improves respiratory function in an animal model experiment in which COPD is induced by CSE.

### [DETAILED DESCRIPTION OF THE EMBODIMENTS]

Hereinafter, the present invention will be described in more detail with reference to examples and experimental examples. However, the scope of the present invention is not intended to be limited by these examples.

### <Example> Improvement activity of asthma, COPD and the like by Epilobium pyrricholophum extract

### 1. Sample preparation

*Epilobium pyrricholophum* extract (extraction site: outpost), *Hypericum erectum* extract extraction site: outpost), *Podocarpus macrophyllus* extract (extraction site: a mixture of the same weight of leaves and flowers), *Artemisia gmelinii* extract (extraction site: outpost), *Poria cocos* extract (extraction site: *Poria cocos* bark) and *Elsholtzia ciliate* extract (extraction site: outpost) were prepared as the samples.

For each extract, 10 times the weight of 70% ethanol was added to the dry powder of each plant part to be extracted, repeatedly extracted twice at 50°C for 6 hours, filtered, concentrated under reduced pressure, and freeze-dried to obtain a powder.

### 2. Experimental method

### 2.1 In vitro experiments

### 2.1.1 NETosis inhibitory activity experiment

HL-60 cells (Human promyelocytic leukemia cells) purchased from the Korea Cell Line Bank were seeded at 2×10⁵ cells/mL in RPMI (10% FBS, 100 U/mL penicillin, 100 mg/mL streptomycin) medium treated with 1% DMSO, incubated in a 37°C, 5% CO₂ cell incubator for 5 days, and differentiated into a mature neutrophil phenotype. After 5 days, the cells were centrifuged at 1,300 rpm for 5 minutes, and treated with 10 mL of HBSS (with Mg⁺, Ca⁺, Hanks' balanced salt solution), which was centrifuged at 1,300 rpm for 5 minutes and washed. The collected cells were diluted with HBSS at a concentration of 1×10⁶ cells/mL, dispensed by 100 µL into 96 well plates, and the samples prepared at each concentration were treated by 50 µL. Then, PMA (phorbol myristate acetate, Sigma) or CSE (cigarette smoke extract) prepared at 400 nM was treated by 50 µL in each well, and then incubated for 3 hours. At this time, CSE was prepared using a standard cigarette 3R4F for research purposes produced by the University of Kentucky. 5 pieces of 3R4F was horizontally mounted on a self-produced cigarette smoke collector and then subjected to burning. Cigarette smoke was collected in 50 mL PBS for 1 minute and 30 seconds with an aspirator, and then adjusted so as to be 0.9-1.0 at OD₃₂₀, which was used for 100% CSE. After 3 hours, 500 nM of Cytox green was treated, and the fluorescence was measured at a wavelength of 485/520 nm (excitation/emission maxima) using a fluorescent plate reader.

### 2.1.2 Evaluation of inhibitory activity of inflammatory cytokines (IL-6 & IL-8) using H292 cell

H292 cell, a bronchial epithelial cell line, was purchased from the Korea Cell Line Bank and used. The H292 cell prepared at a concentration of 2×10⁵ cells/mL in a 24 well plate was subjected to passage culture at 500 µL/well, and when the cell became 80% or more confluent, they were exchanged with serum-free RPMI1640 medium and starvated for 24 hours. Then, using serum-free RPMI1640 medium, stimulating substances (CSE; final 2% or PPE (porcine pancreas elastase, Sigma); final 0.01 U/mL or PM2.5 (fine particulate matter than 2.5 µm, Sigma; final 10 µg /mL) and the sample was diluted to reflect the desired final concentration, and then treated at 500 µL per well. At this time, PPE was dissolved in PBS and frozen stored for use, and PM2.5 was dissolved in DMSO and frozen stored (2 weeks or less) for use. After culturing overnight, the supernatant was collected and used for the measurement of IL-8 and IL-6, and the remaining cells were confirmed for viability by a WST method (water-soluble tetrazolium salt assay). Human IL-8 and IL-6 were measured by an ELISA method, and proceeded according to the protocol of the manufacturer (BD company). At this time, the supernatant for the measurement of IL-8 was diluted 5 times, and the supernatant for the measurement of IL-6 was diluted 40 times and analyzed. After collecting the supernatant, the remaining cells were treated with 500 µL/well of water soluble tetrazolium salt (WST) reagent diluted 1/10 with RPMI1640 + 10% FBS + 1% P/S medium, reacted in a 37°C, 5% CO₂ incubator for 50 minutes, and then the absorbance was measured at 450 nm to confirm the cell viability (WST). Concentrations whose survival rate was lower than that of the control group were excluded from the cytokine analysis.

### 2.1.3 Evaluation of MIP-2 inhibitory activity using MH-S cell

MH-S cell, a mouse alveolar macrophage cell line, was purchased from ATCC, and the composition of the medium used for culture was RMPI-1640 (WELGENE, cat. LM 011-01), 10% FBS (WELGENE, cat. S 001-07), 1% Penicillin-Streptomycin (WELGENE, cat. LS 202-02), and It was 14.3 mM 2-Mercptoethanol (SIGMA). MH-S cell was seeded in a 96 well plate at a concentration of 5×10⁴/well and cultured for 24 hours. After that, the stimulating substance (CSE; final 1% and LPS; final 10 ng/mL) and the sample was diluted with MH-S cell culture medium to reflect the desired final concentration, and treated at 250 uL per well. After culturing for 24 hours, the supernatant was collected and used for the measurement of MIP-2, and the remaining cells were confirmed for viability by a WST method. MIP-2 was measured by an ELISA method, and proceeded according to the protocol of the manufacturer (BD company). After collecting the supernatant, the remaining cells were treated with 200 µL/well of a water soluble tetrazolium salt (WST) reagent diluted 1/10 with MH-S medium, and reacted in a 37°C, 5% CO₂ incubator for 20 minutes, and then the absorbance was measured at 450 nm to confirm the cell viability (WST), and concentrations whose survival rate was lower than that of the control group were excluded from the cytokine analysis.

### 2.1.4 Evaluation of Elastase Inhibitory Activity

Elastase inhibitory activity was measured using the EnzChek® Elastase assay kit (InvitrogenTM). Sample or inhibitor diluted by reflecting the desired final concentration was dispensed by 50 uL into a 96 well plate by reflecting the desired final concentration, and 50 uL of DQ elastin was added, and then the mixture was incubated at 25°C for 1 hour in a dark place. After that, 50 uL of elastase was added, and the enzyme reaction was performed in the dark place for 90 minutes, and the fluorescence intensity was measured under conditions of ex/em=505/515. At this time, the inhibition rate according to the addition of the sample or inhibitor was calculated relatively based on the fluorescence intensity of the sample or the inhibitor-free group (100%).

### 2.2 Evaluation of COPD improvement activity in animal experiments

BALB/C mice (male, 5 weeks old) were purchased from Orient Bio, acclimated for 1 week, and were divided into groups (n=10). In the sample treatment group, 200 mg/kg of the sample was orally administered for 24 days from one week before the start of COPD induction until before dissection, and the same amount of PBS was administered to the untreated sample group (control group) and the COPD group. The Roflumilast administered group, an approved COPD therapeutic agent, was used as a positive control. The Roflumilast group was orally administered at 10 mg/kg daily after the start of COPD induction. For COPD induction, 1.2 units of Porcine Pancreatic Elastase (PPE) and 100% Cigarette Smoke Extraction (CSE) were used. 1.2 unit of PPE was injected three times through intra nasal once every 7 days. CSE was treated with 20 µl through intra nasal for 3 days from the day after PPE treatment. After the last PPE treatment, the dissection was proceeded the next day without CSE treatment. Dissection was performed after anesthetizing with isoflurane using an inhalation anesthesia. During the experiment, feed and drinking water were provided in the form of autonomous food supply, and were reared in an environment of 24°C and 60% humidity.

### 2.2.1 Total number of infiltrating cells in BALF

1 mL of PBS was injected through the airway of the mouse, and then gently massaged to collect 700 µL of bronchoalveolar lavage fluid (BALF). The collected BALF 10 uL was mixed with Accustain T solution in the same amount, and 12 uL was injected into an Accuchip channel, and total cells were automatically counted with a cell counter (ADAM-MC, NANOENTEK. INC, Korea).

### 2.2.2 Analysis of immune cells in BALF through Diff-Quick staining

1 mL of PBS was injected through the airway of the mouse, and then gently massaged to collect 700 µL of bronchoalveolar lavage fluid (BALF). The collected BALF was separated into a supernatant and a cell pellet through centrifugation under conditions of 300×g and 5 minutes. The cell pellet was re-suspended by adding 700 µL of PBS, and 150 µL of this BALF suspension was centrifuged with a Cytospin device (Centrifuge 5403, Eppendorf, Hamburg, Germany) under the conditions of 1000 rpm, 10 min and 4°C, so that BALF cells were attached to the slide. Thereafter, cells were stained according to the protocol of the manufacturer (1-5-1 Wakinohama kaigandori, chuo-ku, Kobe, Japan) using a Diff-Quick staining reagent, and the number of Macrophages, Lympocytes, Neutrophils, and Eosinophils was counted by microscopic observation.

### 2.2.3 Analysis of Cytokines in BALF

1 mL of PBS was injected through the airway of the mouse and then gently massaged to collect 700 µL of bronchoalveolar lavage fluid (BALF). The collected BALF was centrifuged under the conditions of 300×g and 5 minutes, the supernatant was separated, and then used for the analysis of cytokines and chemokines (TARC, KC, MCP-1, MDC, GM-CSF, MIP-2, TNF-α, IL-6, IL-1β, IL-17, IL-10, IFN-y). 30 uL of the supernatant was taken for the analysis of cytokines and chemokines, and then the experiments were performed according to the protocol of the manufacturer of the Q-plex ELISA array kit (Quansis biosciences).

### 2.2.4 Analysis of Cytokines in Lung Tissue

PBS corresponding to 10 times the weight of the lung tissue was added, pulverized with a homogenizer, and then centrifuged under conditions of 12,000 rpm and 20 min. The collected supernatant was used for the analysis of cytokines and chemokines (TARC, KC, MCP-1, MDC, GM-CSF, MIP-2, TNF-α, IL-6, IL-1β, IL-17, IL-10, IFN-y). 30 uL of the supernatant was taken for the analysis of cytokines and chemokines, and then the experiments were performed according to the protocol of the manufacturer of the Q-plex ELISA array kit (Quansis biosciences).

### 2.2.5 Lung Histochemical Examination

Lung tissue was removed from dissected mice, the mesenchyme of the lung was removed, and then fixed to 10% neutral buffered formalin for 3 days. The fixed tissue was dehydrated with ethyl alcohol and xylene, embedded with paraffin, trimmed, and cut to a thickness of 5 um. The prepared slice was attached to a slide, and then stained with H&E and observed under a microscope to confirm the general shape.

### 2.3 Evaluation of the Improvement Activity of Lung Function or Respiratory Function in Animal Experiments

BALB/C, male, 5-week-old mice were purchased from Orient Bio, and acclimated for 1 week, and were divided into groups (n=7). In the sample treatment group, the sample was orally administered at a concentration of 200 mg/kg for 28 days from one week before the start of COPD induction until before dissection, and the same amount of PBS was administered to the negative control group and the COPD group. The Roflumilast administered group was used as a positive control, and the Roflumilast group was orally administered at a concentration of 10 mg/kg once a week for a total of three times after the start of COPD induction. 100% Cigarette Smoke Extraction (CSE) was intraperitoneally administered at 200 µL once a week for a total of 3 times to induce COPD. After completion of the experiment, 350 µL of 1.2% Avertin was intraperitoneally administered, the mice were anesthetized and then dissected. During the experiment, feed and drinking water were provided in the form of autonomous food supply, and were reared in an environment of 24 °C and 60% humidity.

The degree of improvement in respiratory function was measured using Flexi Vent (SCIREQ). A catheter was injected into the airway of an anesthetized mouse, connected to a Flexi Vent device, and then Tissue Damping (G), which is associated with tissue resistance in the Flexi Vent program and reflects the energy dissipation of the alveoli, Tissue Elastnace (H), which is closely related to tissue elasticity and reflects the energy conservation of the alveoli, and Newtonian resistance (Rn) were measured.

### 3. Experiment Results

### 3.1 In vitro Test Results

### 3.1.1 Results of Evaluation of NETosis Inhibitory Activity

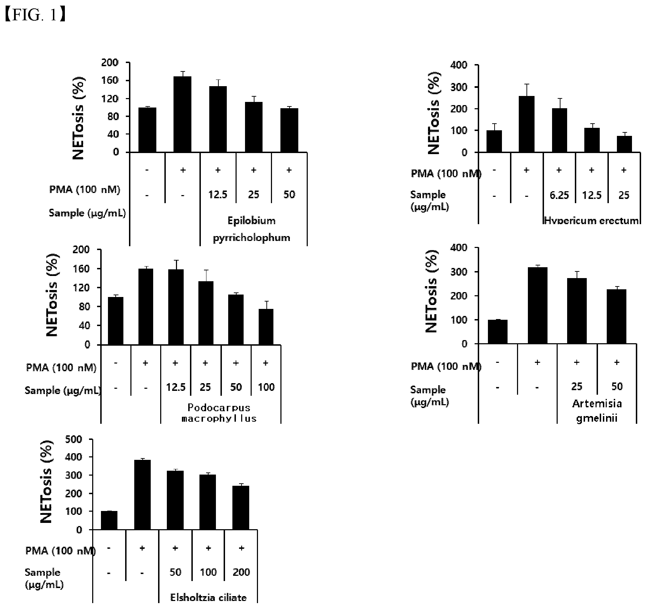

The evaluation results of NETosis inhibitory activities of *Epilobium pyrricholophum* extract, *Hypericum erectum* extract, *Podocarpus macrophyllus* extract, *Artemisia gmelinii* extract and *Elsholtzia ciliate* extract were shown in FIG. 1. Referring to FIG. 1, it can be seen that the *Epilobium pyrricholophum* extract and the like inhibit NETosis in proportion to the treatment concentration.

### 3.1.2 Evaluation Results of Inhibitory Activity of Inflammatory Cytokines (IL-6 & IL-8) using H292 Cells

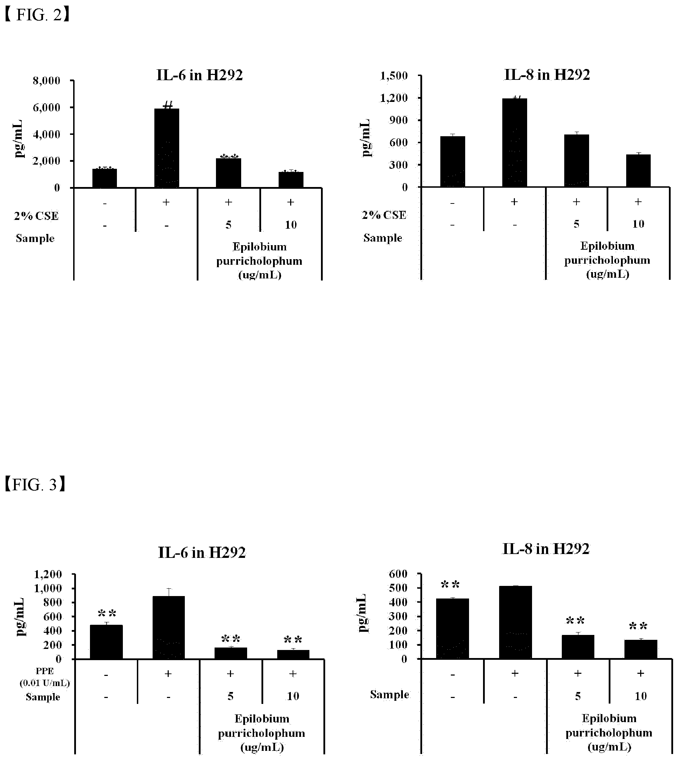

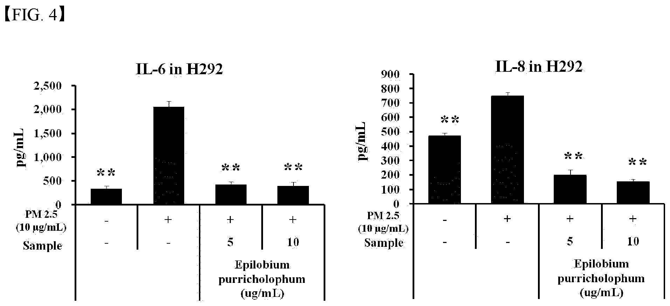

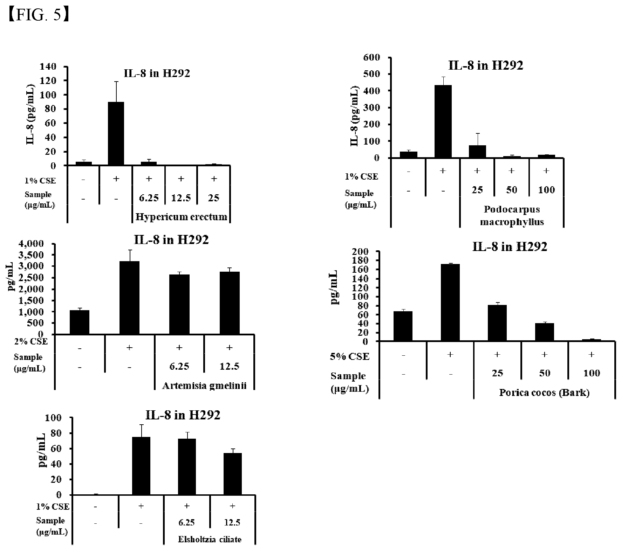

The results of inhibition of the expression of inflammatory cytokines IL-6 and IL-8 of *Epilobium pyrricholophum* extract in H292 cells, which are bronchial epithelial cell lines stimulated by CSE, PPE, and PM2.5, were shown in FIGS. 2 to 4. The results of IL-8 inhibition of *Hypericum erectum* extract, *Podocarpus macrophyllus* extract, *Artemisia gmelinii* extract, *Poria cocos* extract and *Elsholtzia ciliate* extract were shown in FIG. 5. Referring to these results, it can be seen that the *Epilobium pyrricholophum* extract and the like generally inhibit the inflammatory cytokines IL-6 and/or IL-8 in a concentration-dependent manner in H292 cells.

### 3.1.3 Evaluation Results of MIP-2 Inhibitory Activity using MH-S Cells

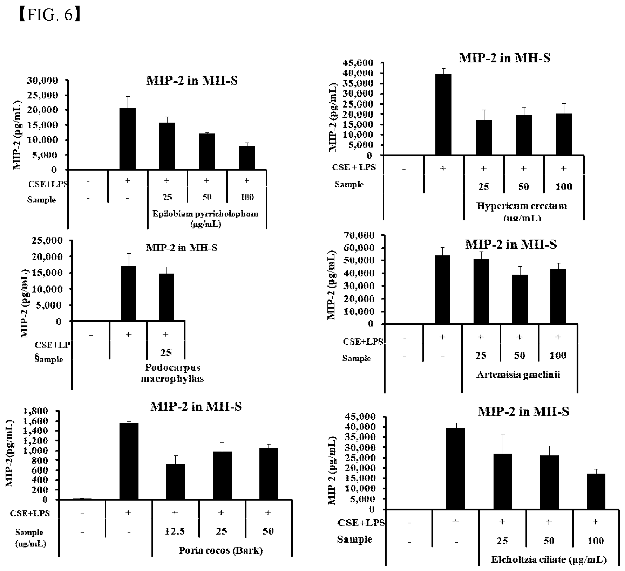

The results are shown in FIG. 6. FIG. 6 shows that the *Epilobium pyrricholophum* extract and the like show MIP-2 inhibitory activity in MH-S cell in a concentration-dependent manner.

### 3.1.4 Evaluation Result of Elastase Inhibitory Activity

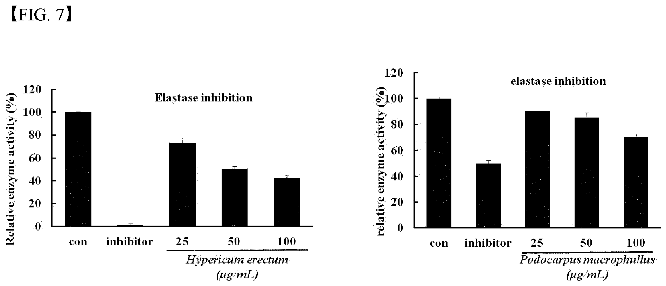

The evaluation results of the Elastase inhibitory activities of the *Hypericum erectum* extract and *Podocarpus macrophyllus* extract were shown in FIG. 7. These samples inhibited Elastase in a concentration-dependent manner.

### 3.2 Results of Evaluation of COPD Improvement Activity in Animal Experiments

### 3.2.1 Measurement Result of Total Number of Infiltrating Cells in BALF

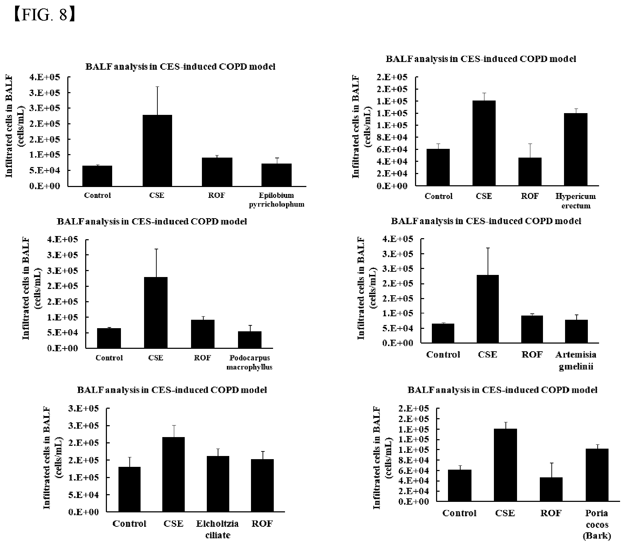

The results of the total number of infiltrating cells in BALF were shown in FIG. 8. *Epilobium pyrricholophum* extract and the like markedly reduced the total number of infiltrating cells in BALF.

### 3.2.2 Results of Analysis of Immune Cells in BALF through Diff-Quick Staining

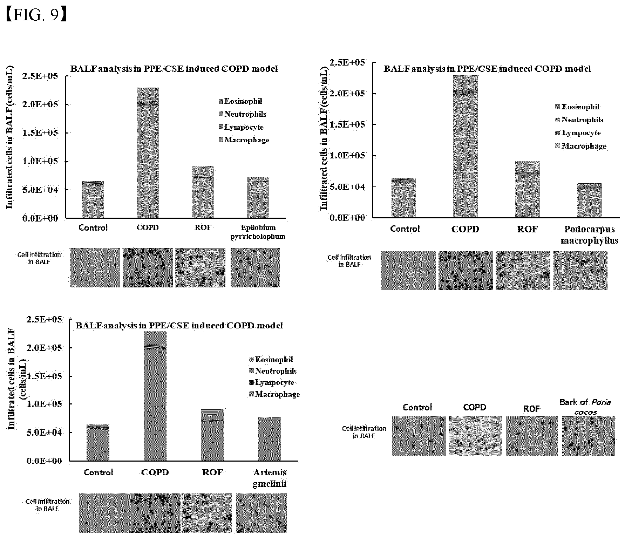

The analysis results of immune cells in BALF of the *Epilobium pyrricholophum* extract, *Podocarpus macrophyllus* extract, *Artemisia gmelinii* extract and *Poria cocos* extract were shown in FIG. 9 together with a micrograph of the number of neutrophil cells infiltrated in BALF. (The *Poria cocos* extract showed only microscopic photographs of neutrophil cell count). These extracts markedly reduced the four immune cells (Eosinophil, Neutrophil, Lympocyte, and Macrophasge) including infiltrating neutrophils in BALF.

### 3.2.3 Analysis Results of Cytokines in BALF

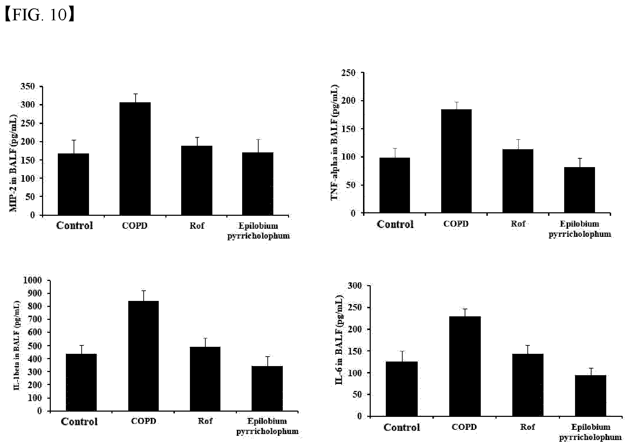

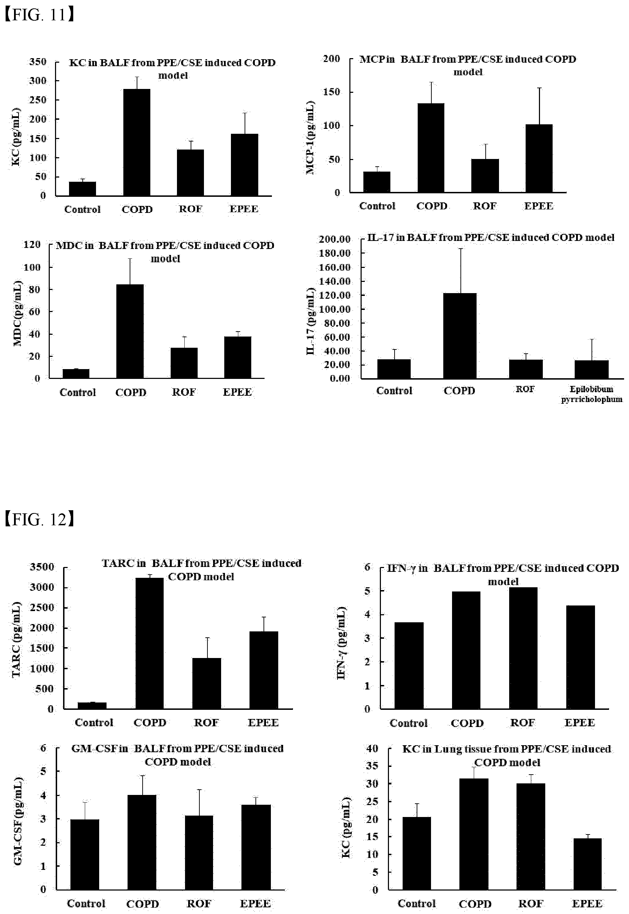

The analysis results of cytokines in BALF of the *Epilobium pyrricholophum* extract were shown in FIGS. 10 to 12. It can be seen that the *Epilobium pyrricholophum* extract lowers the concentration of inflammatory cytokines such as MIP-2, TNF-α, IL-1β, IL-6, KC, MCP-1, IFN-γ of BALF, and lowers the concentration of IL-17, MDC, GM-CSF, etc.

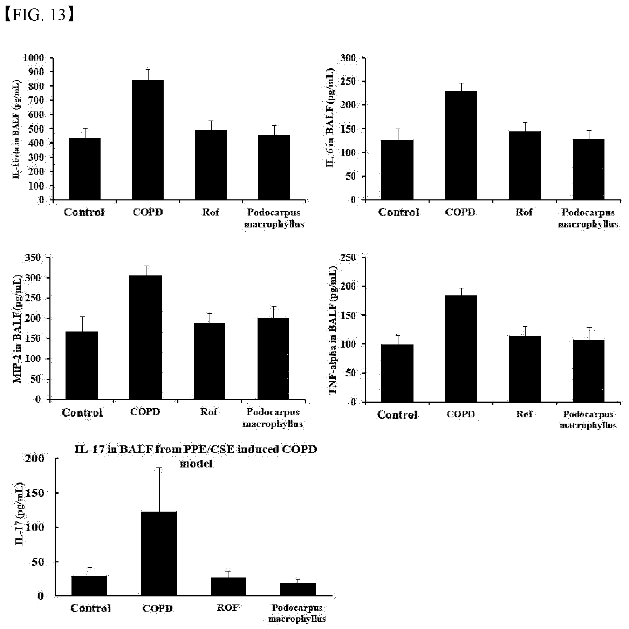

Further, the analysis results of cytokines in BALF of the *Podocarpus macrophyllus* extract were shown in FIG. 13. It shows that the *Podocarpus macrophyllus* extract lowers the concentration of inflammatory cytokines such as IL-1β, MIP-2, IL-6, TNF-α, and IL-17 of BALF.

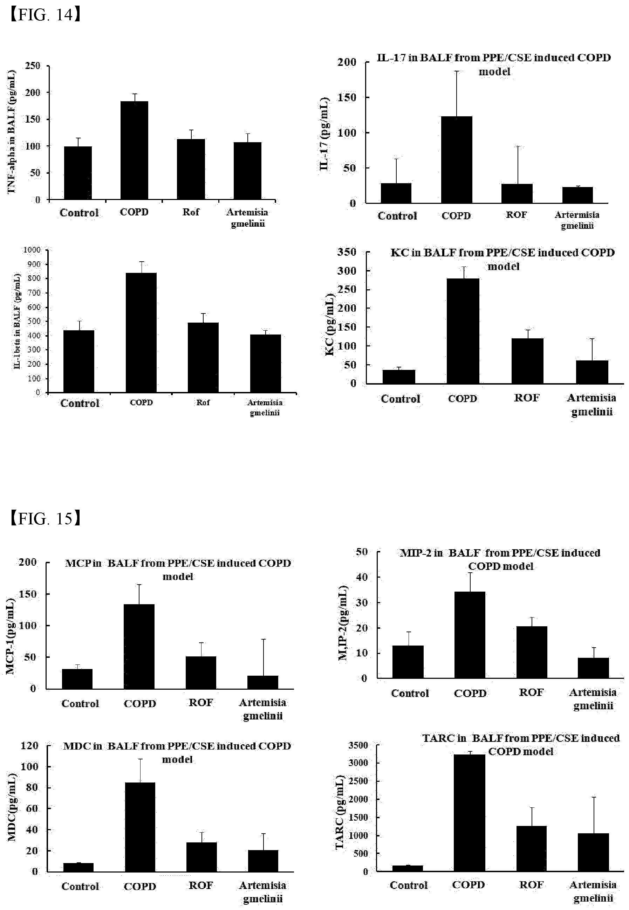

Further, the analysis results of cytokines in BALF of the *Artemisia gmelinii* extract were shown in FIGS. 14 to 16. It can be seen that the *Artemisia gmelinii* extract lowers the concentration of inflammatory cytokines such as TNF-α, IL-17, IL-1β, KC, MCP-1, MDC, MIP-2, TARC, GM-CSF, IFN-γ, IL-6, IL-10, etc. in BALF.

### 3.2.4 Results of Analysis of Cytokines of Lung Tissue

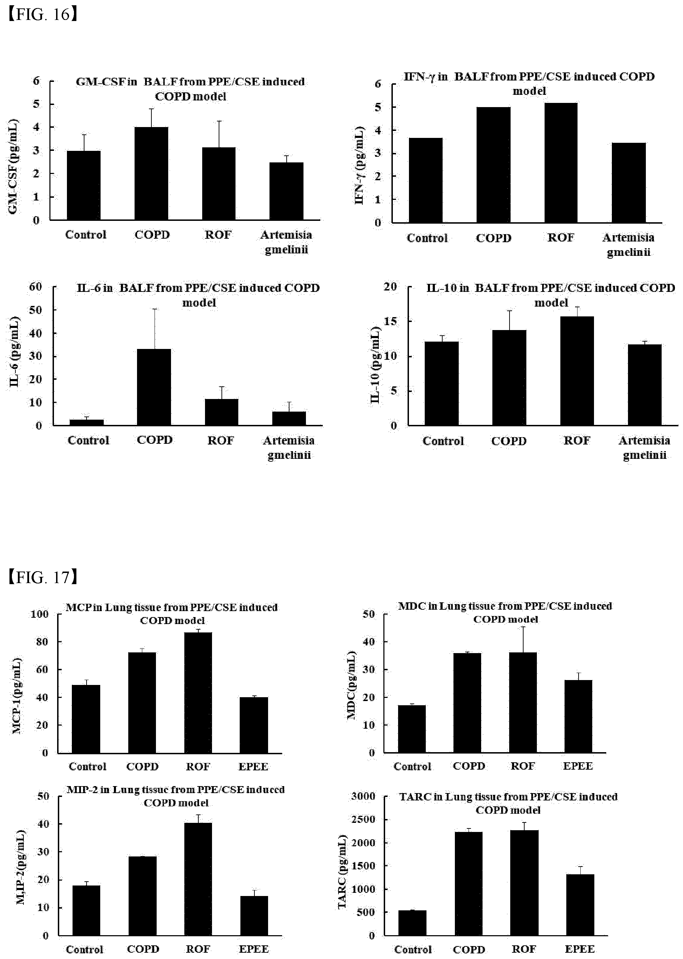

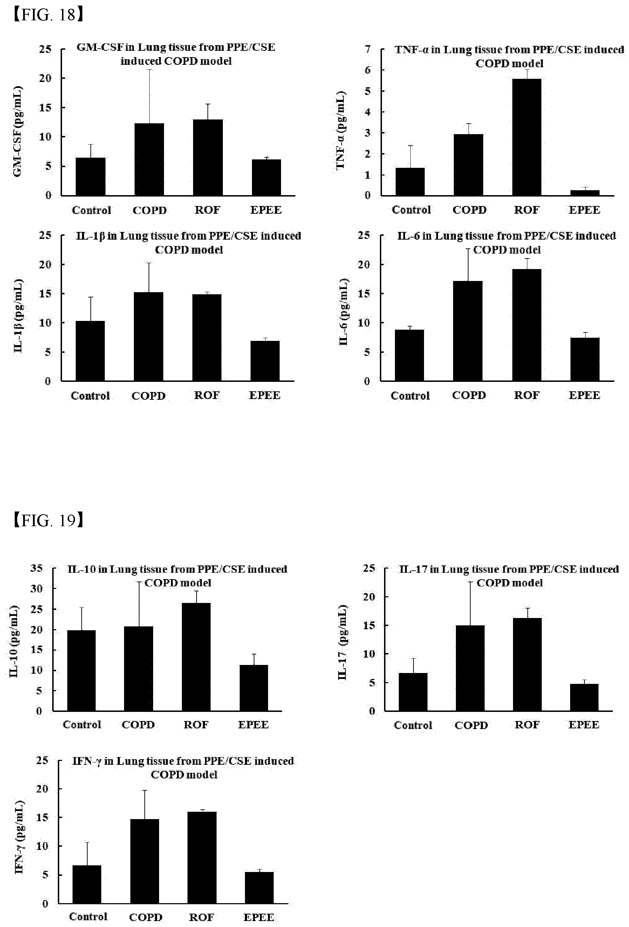

The results of analysis of cytokines in lung tissue of the *Epilobium pyrricholophum* extract were shown in FIGS. 17 to 19. It shows that the *Epilobium pyrricholophum* extract also lowers the concentration of inflammatory cytokines such as MIP-2, TNF-α, IL-1β, IL-6, KC, MCP-1, and IFN-γ in the lung tissue as in the BALF.

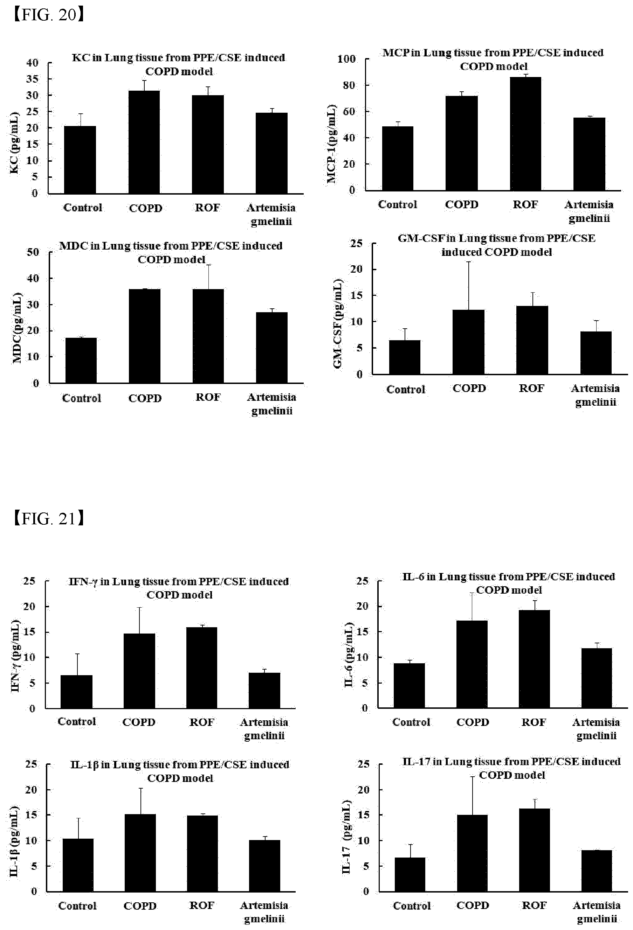

Further, the results of the analysis of the lung tissue cytokines of the *Artemisia gmelinii* extract were shown in FIGS. 20 and 21. It can be seen that the *Artemisia gmelinii* extract lowers the concentration of inflammatory cytokines such as KC, MCP-1, MDC, GM-CSF, IFN-γ, IL-1β, IL-6, and IL-17 in lung tissue.

### 3.2.5 Lung Histochemical Test Results

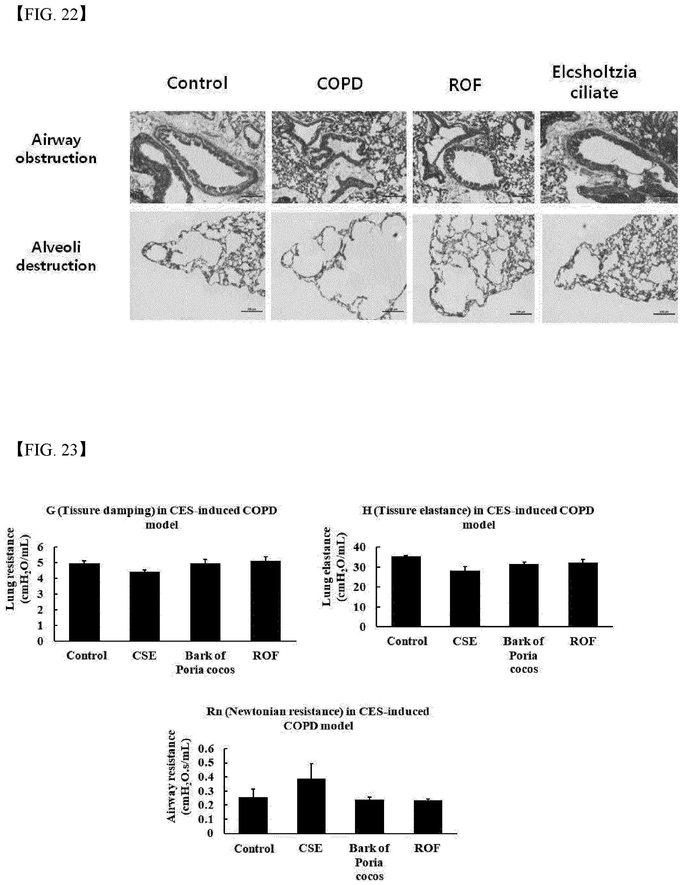

The microscopic photograph of the lung tissue of the *Elsholtzia ciliate* extract was shown in FIG. 22. It shows that the *Elsholtzia ciliate* extract reduces neutrophil infiltration, and restore airway obstruction and alveolar destruction.

### 3.3 Evaluation of Improvement Activity of Lung Function or Respiratory Function in Animal Experiments

The evaluation results of improvement activity of lung function or respiratory function of the *Poria cocos* extract were shown in FIG. 23. FIG. 23 shows that the *Poria cocos* extract activates Tissue Damping (G) and Tissue Elastnace (H) in an animal model in which COPD is induced by CSE, and improves respiratory function by suppressing Newtonian resistance (Rn).

## Claims

1. A composition for ameliorating asthma, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient.

2. The composition as claimed in claim 1,
wherein the composition further comprises *Curcuma longa* extract, *Scutellaria baicalensis* extract, *Trapa japonica* extract, *Perilla frutescens* extract, *Polygonum avivulare* extract, *Rosae multiflorea* extract, *Trigonella foenum* extract, *Piper nigrum* extract or a mixture of two or more thereof as an active ingredient.

3. The composition as claimed in claim 1,
wherein the extract is water, ethanol, or a mixed solvent extract thereof.

4. The composition as claimed in any one of claims 1 to 3,
wherein the composition is a food composition.

5. The composition as claimed in any one of claims 1 to 3,
wherein the composition is a pharmaceutical composition.

6. A composition for ameliorating COPD, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient.

7. The composition as claimed in claim 6,
wherein the composition further comprises *Curcuma longa* extract, *Scutellaria baicalensis* extract, *Trapa japonica* extract, *Perilla frutescens* extract, *Polygonum avivulare* extract, *Rosae multiflorea* extract, *Trigonella foenum* extract, *Piper nigrum* extract or a mixture of two or more thereof as an active ingredient.

8. The composition as claimed in claim 6,
wherein the extract is water, ethanol, or a mixed solvent extract thereof.

9. The composition as claimed in any one of claims 6 to 8,
wherein the composition is a food composition.

10. The composition as claimed in any one of claims 6 to 8,
wherein the composition is a pharmaceutical composition.

11. A composition for ameliorating inflammatory lung diseases, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient.

12. The composition as claimed in claim 11,
wherein the composition further comprises *Curcuma longa* extract, *Scutellaria baicalensis* extract, *Trapa japonica* extract, *Perilla frutescens* extract, *Polygonum avivulare* extract, *Rosae multiflorea* extract, *Trigonella foenum* extract, *Piper nigrum* extract or a mixture of two or more thereof as an active ingredient.

13. The composition as claimed in claim 11,
wherein the extract is water, ethanol, or a mixed solvent extract thereof.

14. The composition as claimed in any one of claims 11 to 13,
wherein the composition is a food composition.

15. The composition as claimed in any one of claims 11 to 13,
wherein the composition is a pharmaceutical composition.

16. A composition for improving lung function and respiratory function, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient.

17. The composition as claimed in claim 16,
wherein the composition further comprises *Curcuma longa* extract, *Scutellaria baicalensis* extract, *Trapa japonica* extract, *Perilla frutescens* extract, *Polygonum avivulare* extract, *Rosae multiflorea* extract, *Trigonella foenum* extract, *Piper nigrum* extract or a mixture of two or more thereof as an active ingredient.

18. The composition as claimed in claim 16,
wherein the extract is water, ethanol, or a mixed solvent extract thereof.

19. The composition as claimed in any one of claims 16 to 18,
wherein the composition is a food composition.

20. The composition as claimed in any one of claims 16 to 18,
wherein the composition is a pharmaceutical composition.

21. A composition for ameliorating respiratory diseases caused by smoking or fine dust, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient.

22. The composition as claimed in claim 21,
wherein the respiratory disease is asthma, COPD, diffuse interstitial lung disease, acute respiratory distress syndrome or acute lung injury.

23. The composition as claimed in claim 21,
wherein the composition further comprises *Curcuma longa* extract, *Scutellaria baicalensis* extract, *Trapa japonica* extract, *Perilla frutescens* extract, *Polygonum avivulare* extract, *Rosae multiflorea* extract, *Trigonella foenum* extract, *Piper nigrum* extract or a mixture of two or more thereof as an active ingredient.

24. The composition as claimed in claim 21,
wherein the extract is water, ethanol, or a mixed solvent extract thereof.

25. The composition as claimed in any one of claims 21 to 24,
wherein the composition is a food composition.

26. The composition as claimed in any one of claims 21 to 24,
wherein the composition is a pharmaceutical composition.

27. A composition for improving lung damage caused by smoking or fine dust, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient.

28. The composition as claimed in claim 27,
wherein the composition further comprises *Curcuma longa* extract, *Scutellaria baicalensis* extract, *Trapa japonica* extract, *Perilla frutescens* extract, *Polygonum avivulare* extract, *Rosae multiflorea* extract, *Trigonella foenum* extract, *Piper nigrum* extract or a mixture of two or more thereof as an active ingredient.

29. The composition as claimed in claim 27,
wherein the extract is water, ethanol, or a mixed solvent extract thereof.

30. The composition as claimed in any one of claims 27 to 29,
wherein the composition is a food composition.

31. The composition as claimed in any one of claims 27 to 29,
wherein the composition is a pharmaceutical composition.

32. A composition for ameliorating lung diseases associated with NETosis, which comprises *Epilobium pyrricholophum* extract, *Hypericum erectum* extract, *Podocarpus macrophyllus* extract, *Artemisia gmelinii* extract, *Poria cocos* extract or *Elsholtzia ciliate* extract as an active ingredient.

33. The composition as claimed in claim 32,
wherein the lung disease associated with NETosis is asthma, cystic fibrosis, respiratory syncytial virus (RSV) bronchiolitis, influenza virus infection, bacterial pneumonia, tuberculosis, or transfusion-related acute lung injury.

34. The composition as claimed in claim 32,
wherein the composition further comprises *Curcuma longa* extract, *Scutellaria baicalensis* extract, *Trapa japonica* extract, *Perilla frutescens* extract, *Polygonum avivulare* extract, *Rosae multiflorea* extract, *Trigonella foenum* extract, *Piper nigrum* extract or a mixture of two or more thereof as an active ingredient.

35. The composition as claimed in claim 32,
wherein the extract is water, ethanol, or a mixed solvent extract thereof.

36. The composition as claimed in any one of claims 32 to 35,
wherein the composition is a food composition.

37. The composition as claimed in any one of claims 32 to 35,
wherein the composition is a pharmaceutical composition.
